# EUROPÄISCHE PATENTSCHRIFT

(11) **EP 0 451 345 B1**
(45) Veröffentlichungstag und Bekanntmachung des Hinweises auf die Patenterteilung: **21.02.1996**
(21) Anmeldenummer: 90123135.7
(22) Anmeldetag: 03.12.1990
(51) Int. Cl.: G01N 1/22, G01N 1/28

(54) **Vorrichtung zur Vorbereitung von Gasproben für die Analyse**
Apparatus for preparing gas samples for analysis
Appareil pour la préparation des échantillons de gaz pour analyse

(30) Priorität: 11.04.1990 DE 9004227 U
(43) Veröffentlichungstag der Anmeldung: 16.10.1991
(73) Patentinhaber: SIEMENS AKTIENGESELLSCHAFT, D-80333 München (DE)
(72) Erfinder: Aidam, Eckhard, W-6729 Jockgrim (DE); Wendt, Klaus, Dr., W-7500 Karlsruhe 21 (DE); Meister, Günter, W-7514 Eggenstein (DE)

(56) Entgegenhaltungen:
- DE-A- 3 741 390
- DE-B- 2 029 959
- GB-A- 1 527 003
- SOVIET INVENTIONS ILLUSTRATED, Section El, Woche 8712, 6. Mai 1987 DERWENT PUBLICATIONS LTD., London, SO3
- PATENT ABSTRACTS OF JAPAN, unexamined applications, C Field, Band 10, Nr. 94,11.April 1986 THE PATENT OFFICE JAPENESE GOVERNMENT Seite 80 C 338

## Beschreibung

Die Erfindung bezieht sich auf eine Einrichtung in Aggregaten zur Meßgasaufbereitung für die Gasanalyse.

Komponenten von Gasgemischen, insbesondere Verbrennungsabgasen, werden mit Gasanalysegeräten gemessen, denen das zu analysierende Gas staub- und kondensatfrei zugeführt werden muß. Bekannte Aufbereitungsaggregate bestehen aus einer beheizten Entnahmesonde, einer ebenfalls beheizten Entnahmeleitung, Gaskühlern mit Kondensatabführung, Gaspumpen, Feinfiltern und Vorrichtungen zur Einstellung der Strömungsgeschwindigkeit in den Gasanalysegeräten.

Werden die gasführenden Teile nach dem Gaskühler nicht beheizt, kann sich in vielen Fällen bei Taupunktunterschreitung ein Aerosol- bzw. Säureniederschlag bilden, der in relativ kurzen Zeitabständen aus Gasleitungen, Filtern, Gaspumpen sowie den Analysegeräten entfernt werden muß. Somit entsteht ein erheblicher Wartungsaufwand, dies trifft insbesondere bei Meßeinrichtungen zur Überwachung von Verbrennungsabgasen mit hohem SO₃-Anteil zu.
Zur Vermeidung derartiger Aerosolabscheidungen müssen Aggregate und Gasleitungen nach dem Gaskühler auf Temperaturen = 70 °C beheizt werden, was mit hohen Kosten verbunden ist.

In DE-A-37 41 390 ist ein Probenahmesystem zur Bestimmung des SO₃- und NH₃-Gehaltes von Rauchgasen beschrieben, bei dem das Meßgas über ein Staubfilter, das aus einer mit Quarzglaswatte gestopften Glashülse besteht, einem Kondensator zugeführt wird, auf dessen gekühlten Wänden sich Ammoniumverbindungen und Schwefelsäure niederschlagen. Zum Entfernen etwaiger Ammoniom- und Schwefelsäure-Aerosole wird das aus dem Kondensator austretende Gas durch ein Aerosolfilter in Form eines Quarzfaserfilters geleitet.

Aus DE-B-20 29 959 ist eine Vorrichtung zum Entfernen von Wasser und festen Partikeln aus einem zu analysierenden Gas bekannt, in der das Gas einen Kondensator und anschließend einen Wasserabscheider durchströmt, der aus einem senkrechten, teilweise mit Glaskugeln gefülltem Zylinder besteht und die im Kondensator entstandenen Wassertropfen aus dem Gasstrom zurückhält.

Der vorliegenden Erfindung liegt die Aufgabe zugrunde, eine Einrichtung zu schaffen, welche störende Aerosole wirksam aus dem Meßgas entfernt und damit die aus dem Niederschlag von Aerosolen herrührenden Beeinträchtigungen in den Gas führenden Teilen weitgehend unterbindet.

Erfindungsgemäß wird diese Aufgabe mit den im Anspruch 1 angegebenen Maßnahmen gelöst.

Mit dem erfindungsgemäß eingebauten Aerosolabschelder wird erreicht, daß im Temperaturbereich des Säuretaupunkts die Strömungsgeschwindigkeit des Gases erheblich herabgesetzt wird, wodurch sich bei langsamer Abkühlung große Kolloidteilchen bzw. Aerosole bilden, die sich unmittelbar nach ihrer Entstehung an der großen Oberfläche der Granulatfüllung abscheiden, so daß keine Aerosole mehr in die auf den Aerosolabscheider folgenden Teile der Gasaufbereitungsaggregate und der Analysegeräte gelangen. Ein Gasaufbereitungsaggregat mit diesem Aerosolabscheider erfordert nur einen geringen Wartungsaufwand, da die abgeschiedene Säure tropfenweise durch die Gasleitung bei Umgebungstemperatur zum Kondensatgefäß im Gaskühler transportiert wird. Eine Beheizung der gasführenden Teile des Aufbereitungsaggregats nach dem Gaskühler ist nicht mehr notwendig.

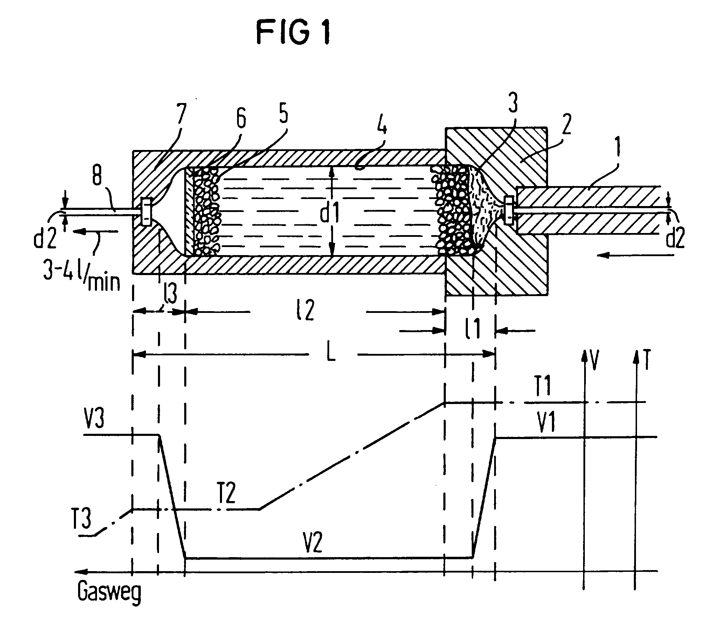

Zur Erläuterung der Erfindung ist in der Figur 1 ein Ausführungsbeispiel eines Aerosolabscheiders schematisch dargestellt, mit einem Diagramm, welches den Verlauf der Gastemperaturen und -strömungsgeschwindigkeiten über die Länge des Gaswegs im Abscheider veranschaulicht.

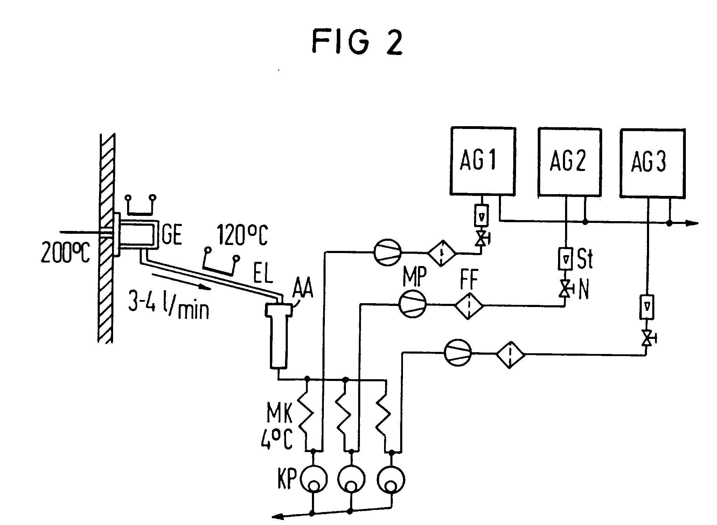

Figur 2 zeigt eine Überwachungsanlage für Verbrennungsabgase mit Gasentnahme-, Gasaufbereitungsaggregraten und Gasanalysegeräten.

Bei dem Aerosolabscheider in Figur 1 mündet eine beheizte Meßgaszuleitung 1 in ein zylindrisches Gefäß 4 aus Quarzglas, welches mit einem inerten Granulat 5, vorzugsweise Quarzglasgranulat, gefüllt ist. Der innere Durchmesser dl des Gefäßes 4 ist um ein Mehrfaches größer als die Durchmesser d2 der Zuleitung 1 und der Ableitung 8 am unteren Ende des Gefäßes 4. Daraus ergibt sich, daß die Querschnitte von Zu- und Ableitung 1, 8 um zwei dezimale Größenordnungen kleiner sind als der Querschnitt des Gefäßes 4. Die Länge L des Gefäßes 4 und die Körnung des Granulats 5 sind derart bemessen, daß die mittlere Strömungsgeschwindigkeit V2 des Gases im Gefäß 4 um mindestens eine dezimale Größenordnung kleiner ist als die Strömungsgeschwindigkeit V1 in der Zu- bzw. V3 in der Ableitung und das Meßgas bis deutlich unter den Säuretaupunkt von Schwefelsäure (H₂SO₄) abgekühlt wird. Aus dem rechts dargestellten Diagramm sind die Gasgeschwindigkeiten und die Gastemperaturen über den Gasweg im Aerosolabscheider aufgetragen. Damit die Gasabkühlung mit Sicherheit innerhalb des Bereichs geringer Strömungsgeschwindigkeit beginnt, ist das obere Ende des Gefäßes 4 im Bereich des Anschlusses der Gaszuleitung 1 mit Hilfe einer Heizmanschette, die sich über die Teillänge l1 erstreckt, beheizt, so daß der Temperaturabfall erst bei kleiner Strömungsgeschwindigkeit einsetzt. Direkt hinter der Einmündung der Gaszuleitung 1 befindet sich eine Schicht von Quarzwolle 3. Am unteren Ende des Gefäßes 4 ist eine Quarzglasfritte 6 angeordnet.

In dem gezeigten Beispiel beträgt der Durchmesser des Gefäßes 4 70 mm, die Länge l2 450 mm, die Länge l1 und l3 je 100 mm. Die Strömungsmenge beträgt 3 bis 4 Liter pro Minute. Die Temperatur T1 in der beheizten Zuleitung 1 liegt zwischen 100 und 120°. Das Gas wird im Aerosolabscheider bis auf die Temperatur T2 von ca. 45° abgekühlt und bei Umgebungstemperatur T3 über die Ableitung 8 zu den Aggregaten der Gasaufbereitung geführt.

Bei Messung der Schadstoffkomponente Schwefeldioxyd ist darauf zu achten, daß Kondensatflüssigkeit im unteren Endabschnitt des Gefäßes 4 und der Ableitung 8 nicht mit dem zu analysierenden Gasgemisch in Berührung kommt, weil Schwefeldioxyd darin wieder gelöst wird, was zu Verfälschungen der Messung führt. Um dies zu vermeiden, wird ein Teil des Gefäßes 4 im Bereich der Niedrigtemperatur, also etwa auf der Länge l3, mit einer Zusatzbeheizung 7 auf eine Temperatur über dem Kondensattaupunkt beheizt. Die Funktion des Aerosolabscheiders wird dadurch nicht gestört, weil der Kondensattaupunkt mindestens 30 °C unter dem Säuretaupunkt von Schwefelsäure liegt.

In der in Figur 2 dargestellten Meßanlage für die Bestimmung von Schadstoffkomponenten in Verbrennungsabgasen wird das etwa 200 °C heiße Meßgas mittels der Gasentnahme GE und der auf 120 °C beheizten, relativ kurzen Entnahmeleitung EL entnommen und dem Eingang des Aerosolabscheiders AA zugeführt. Auf diesen folgen Gaskühler MK, in welchen das Meßgas auf etwa 4 °C abgekühlt wird. Das dabei entstehende Kondensat wird über Kondensatpumpen KP abgeführt. Über Meßgaspumpen MP, Feinfilter FF und Strömungsmesser ST mit Nadelventilen N wird das aufbereitete Meßgas Gasanalysegeräten AG1, AG2 und AG3 zugeführt, die jeweils eine der Meßgaskomponenten messen.

## Patentansprüche

1. Einrichtung zur Meßgasaufbereitung von Verbrennungsgasen mit SO₃-Anteil für die Gasanalyse mit einem Aerosolabscheider (AA), einer beheizten Gaszuleitung (1) und einer nichtbeheizten Gasableitung (8), **dadurch gekennzeichnet**, daß der Aerosolabscheider (AA) aus einem aus säurebeständigem Material bestehenden, zylindrischen Gefäß (4) besteht, das mit inertem Granulat gefüllt ist, das ein oberes Ende aufweist, welches an die Gaszuleitung angeschlossen ist und das ein anderes Ende aufweist, an welches die Gasableitung (8) angeschlossen ist, und daß der Durchmesser des Gefäßes (4) ein solches Mehrfaches der Durchmesser (d2, d3) der Gaszu- und der Gasableitung beträgt und die effektive Länge des Gefäßes (4) und die Körnung des Granulats derart bemessen sind, daß die mittlere Strömungsgeschwindigkeit (V2) des Meßgases im Gefäß (4) um mindestens eine dezimale Größenordnung kleiner ist als die Strömungsgeschwindigkeiten (V1, V3) in den Zu- und Ableitungen (1, 8), und daß das Meßgas im Gefäß (4) bis deutlich unter den Taupunkt von Schwefelsäure abgekühlt wird.

2. Einrichtung nach Anspruch 1, **dadurch gekennzeichnet**,daß die Querschnitte von Zuleitung (1) und Ableitung (8) um zwei dezimale Größenordnungen kleiner sind als der Querschnitt des Gefäßes (4).

3. Einrichtung nach Anspruch 1, **dadurch gekennzeichnet**, daß das zylindrische Gefäß (4) aus Quarzglas und die Füllung aus Quarzglasgranulat besteht.

4. Einrichtung nach Anspruch 1, **gekennzeichnet durch** eine Einrichtung (2) zur Beheizung des oberen Endabschnitts des Gefäßes (4).

## Claims

1. Device for preparing combustion gases, which have an SO₃-content, as measurement gas for gas analysis, having an aerosol separator (AA), a heated gas supply line (1) and an unheated gas outlet line (8), characterised in that the aerosol separator (AA) consists of a cylindrical receptacle (4), which consists of acid-proof material, is filled with inert granular material, has an upper end which is connected to the gas supply line, and has another end to which is connected the gas outlet line (8), and in that the diameter of the receptacle (4) amounts to such a multiple of the diameters (d2, d3) of the gas supply line and of the gas outlet line, and the effective length of the receptacle (4) and the particle size of the granular material are dimensioned in such a way that the average flow velocity (V2) of the measurement gas in the receptacle (4) is lower than the flow velocities (V1, V3) in the supply line and outlet line (1, 8) by at least one decimal order of magnitude, and in that the measurement gas is cooled in the receptacle (4) to distinctly below the dew point of sulphuric acid.

2. Device according to claim 1, characterised in that the cross sections of supply line (1) and outlet line (8) are smaller than the cross section of the receptacle (4) by two decimal orders of magnitude.

3. Device according to claim 1, characterised in that the cylindrical receptacle (4) consists of quartz glass and the filling consists of quartz glass granules.

4. Device according to claim 1, characterised by a device (2) for heating the upper end section of the receptacle (4).

## Revendications

1. Dispositif de préparation de gaz de combustion à mesurer ayant un pourcentage de SO₃, pour l'analyse des gaz, comprenant un séparateur d'aérosol (AA), une canalisation chauffée d'amenée de gaz (1) et une canalisation non chauffée d'évacuation de gaz (8), caractérisé par le fait que le séparateur d'aérosol (1) est constitué d'une enceinte (4) cylindrique en un matériau résistant aux acides, qui est emplie de granulé inerte, et qui a une extrémité supérieure raccordée à la canalisation d'amenée de gaz, et une autre extrémité raccordée à la canalisation d'évacuation de gaz (8), et que le diamètre de l'enceinte (4) est égal à un multiple tel des diamètres (d2, d3) des canalisations d'amenée et d'évacuation de gaz et que la longueur effective de l'enceinte(4) et la taille des granulés sont telles que la vitesse moyenne d'écoulement (V2) du gaz à mesurer dans l'enceinte (4) est inférieure, au moins d'un ordre de grandeur décimal, aux vitesses d'écoulement (V1,V3) dans les canalisations d'amenée et de sortie (1,8), et que le gaz à mesurer est refroidi dans l'enceinte (4) jusqu'à une température nettement inférieure au point de rosée de l'acide sulfurique.

2. Dispositif suivant la revendication 1, caractérisé par le fait que les sections transversales de la canalisation d'amenée (1) et de la canalisation d'évacuation (8) sont inférieures, de deux ordres de grandeur décimaux, à la section transversale de l'enceinte(4).

3. Dispositif suivant la revendication 1, caractérisé par le fait que l'enceinte cylindrique (4) est en verre au quartz et que le remplissage est en granulé de verre au quartz.

4. Dispositif suivant la revendication 1, caractérisé par un dispositif (2) pour chauffer la partie d'extrémité supérieure de l'enceinte(4).
